(19) Europäisches Patentamt

European Patent Office

Office européen des brevets

(11) **EP 0 813 858 B1**

(12) **FASCICULE DE BREVET EUROPEEN**

(45) Date de publication et mention
de la délivrance du brevet:
**25.11.1998 Bulletin 1998/48**

(51) Int. Cl.$^6$: **A61K 7/043**

(21) Numéro de dépôt: **97401115.7**

(22) Date de dépôt: **21.05.1997**

(54) **Utilisation de l'acide silicique colloidal dans une composition de vernis-à-ongles**

Verwendung von kolloidaler Kieselsäure in einer Nagellackzusammensetzung

Use of colloidal silica in a nail varnish composition

(84) Etats contractants désignés:
**DE ES FR GB IT**

(30) Priorité: **19.06.1996 FR 9607636**

(43) Date de publication de la demande:
**29.12.1997 Bulletin 1997/52**

(73) Titulaire: **L'OREAL**
**75008 Paris (FR)**

(72) Inventeurs:
• **Ramin, Roland**
**91760 Itteville (FR)**
• **Toumi, Béatrice**
**95880 Enghien (FR)**
• **Cazeneuve, Colette**
**75015 Paris (FR)**

(74) Mandataire: **Dodin, Catherine**
**L'Oreal-D.P.I.,**
**90, rue du Général Roguet**
**92583 Clichy Cédex (FR)**

(56) Documents cités:
**EP-A- 0 193 717** **EP-A- 0 504 754**
**EP-A- 0 714 653** **EP-A- 0 745 372**
**US-A- 4 439 494** **US-A- 5 071 639**

• **DATABASE WPI Section Ch, Week 9624 Derwent Publications Ltd., London, GB; Class A96, AN 96-235922 XP002027075 & JP 08 092 038 A (KAO CORP) , 9 avril 1996**
• **PATENT ABSTRACTS OF JAPAN vol. 009, no. 130 (C-284), 5 juin 1985 & JP 60 016910 A (SHISEIDO KK), 28 janvier 1985,**

**Description**

La présente invention a trait à l'utilisation d'acide silicique colloïdal dans une composition de vernis à ongles en milieu solvant ou aqueux.

On connaît des compositions à appliquer par exemple sur l'ongle, de type vernis à ongles ou base de soin pour ongles en milieu solvant, comprenant de manière usuelle, au moins un polymère filmogène, éventuellement un agent plastifiant, des pigments, des agents rhéologiques et des solvants.

Une telle composition peut ainsi permettre d'embellir l'ongle. Toutefois, les compositions connues jusqu'à présent présentent le défaut de s'user rapidement et de s'écailler facilement, ce qui contraint l'utilisatrice à renouveler à des intervalles de temps très rapprochés, l'application d'une nouvelle couche de vernis après élimination de la couche abîmée. Ce défaut des bases de soin pour ongles et des vernis à ongles a conduit la demanderesse à rechercher des agents qui, incorporés dans une composition de vernis ou de base de soin pour les ongles, conféreraient à celles-ci un plus grand pouvoir amortissant des chocs et, par conséquent, augmenteraient leur résistance à l'écaillage ainsi que leur résistance aux chocs.

La demanderesse a constaté que, de manière surprenante, l'acide silicique colloïdal pouvait être utilisé de manière satisfaisante pour améliorer la résistance aux chocs et à l'écaillage des vernis et bases de soin pour l'ongle, ce qui a pour conséquence l'amélioration de leur tenue et de leur résistance à l'usure.

Le document JP8092038 décrit une composition aqueuse à appliquer sur l'ongle comprenant une émulsion d'un polymère et de la silice colloïdale. De telles compositions présentent une résistance élevée à l'abrasion. Toutefois, la fonction d'agent amortissant de l'acide silicique colloïdal, tout comme l'amélioration de la résistance aux chocs et à l'écaillage, ne sont ni mentionnés ni suggérés par ce document.

Ainsi, la présente invention a pour objet l'utilisation de l'acide silicique colloïdal en tant qu'agent amortissant dans une composition de vernis ou de base de soin pour ongles comprenant au moins une matière filmogène et au moins un solvant organique et/ou aqueux.

On entend dans la présente description par 'agent amortissant', un agent susceptible d'améliorer la résistance des vernis sur l'ongle aux chocs et/ou à l'écaillage, ce qui a pour conséquence une amélioration de la tenue des vernis sur l'ongle et/ou leur résistance à l'usure.

L'utilisation d'une composition selon l'invention sur les ongles permet donc l'obtention d'un film de vernis à ongles ou de base de soin qui présente une meilleure résistance et par conséquent, une plus grande durée de vie.

La composition selon l'invention comprend donc au moins une matière filmogène. Lorsque la base de soin ou le vernis selon l'invention est une composition en milieu solvant organique la matière filmogène peut être choisie, notamment, parmi les résines alkydes, acryliques et/ou vinyliques, les polyuréthannes et les polyesters, les celluloses et dérivés cellulosiques telles que la nitrocellulose, et les résines résultant de la condensation de formaldéhyde avec une arylsulfonamide, et leurs mélanges.

Dans ce cas, la matière filmogène est généralement en solution, par exemple à 5-25% en poids, dans un solvant organique tel qu'un hydrocarbure aromatique comme par exemple le toluène ou le xylène, un hydrocarbure aliphatique comme par exemple le n-heptane, un ester comme par exemple l'acétate d'éthyle, l'acétate de butyle, une cétone, comme par exemple l'acétone, la méthyl éthyl cétone, un alcool, comme par exemple l'éthanol, l'isopropanol et le butanol, et les éthers de glycol ainsi que leurs mélanges.

Lorsque la base de soin ou le vernis à ongles selon l'invention est une composition en milieu aqueux, la composition peut comprendre le polymère sous forme de particules de polymère filmogène en dispersion.

Parmi les polymères filmogènes susceptibles d'être utilisés, on peut citer les polyuréthannes, par exemple anioniques, les polyesters-polyuréthannes, les polyéther-polyuréthannes, les polymères radicalaires notamment de type acrylique, acrylique styrène et/ou vinylique, les polyesters, les résines alkydes, seuls ou en mélange.

La dispersion peut également comprendre un polymère associatif de type polyuréthanne ou une gomme naturelle telle que la gomme xanthane. La composition présente, de préférence, un taux de matière sèche de 25-45% en poids.

La composition peut également comprendre un agent plastifiant et éventuellement des agents rhéologiques.

Parmi les agents plastifiants, on peut citer les citrates, les phtalates, les esters et/ou le camphre, généralement utilisés en une quantité de 5-30% en poids par rapport au poids de la composition. De préférence, on utilise un citrate tel que l'acétyl tributyl citrate.

Parmi les agents rhéologiques, on peut citer les bentonites organophiles, les dérivés de cellulose, les dérivés d'acide polyacrylique réticulé, les gommes de guar ou de caroube, ainsi que les gommes de xanthane.

L'acide silicique colloïdal susceptible d'être utilisé dans la composition selon l'invention est une silice pyrogénée ou traitée en surface, qui peut se présenter sous forme de silice pyrogénée hydrophile, de silice pyrogénée hydrophobe, ou de silice traitée en surface par un traitement organique.

Les silices pyrogénées peuvent être obtenues par hydrolyse à haute température d'un composé volatil du silicium dans une flamme oxhydrique. Il s'ensuit la production d'une silice finement divisée. Par réaction, on peut modifier chimiquement la surface de ladite silice, par réduction du nombre de groupes silanols afin d'obtenir une silice hydrophobe.

L'acide silicique colloïdal présente de préférence une taille des particules pouvant être nanométrique à micrométrique, par exemple de l'ordre de 10-200 nm. L'acide silicique colloïdal selon l'invention peut être entre autre choisi parmi les composés vendus par DEGUSSA, sous les marques Aerosil MOX80 ou Aerosil COK84 qui sont des silices spéciales, Aerosil R972 qui est une silice hydrophobe, ou encore Aerosil OK412 qui est une silice traitée en surface ou sous la marque Aerosil 200 qui est une silice hydrophile.

La composition selon l'invention peut comprendre l'acide silicique colloïdal en une quantité de 0,05 % à 5% en poids, de préférence en une quantité de 0,5 - 2 % en poids par rapport au poids total de la composition.

La composition selon l'invention peut en outre comprendre tout additif connu de l'homme du métier comme étant susceptible d'être incorporé dans une telle composition, tels que des agents d'étalement, des agents mouillants, des agents dispersants, des anti-mousses, des conservateurs, des filtres UV, des colorants, des pigments, des nacres, des actifs tels que le N-butylformal, le D-panthénol, le phytantriol, les vitamines et leurs dérivés, la kératine et ses dérivés, la mélanine, le collagène, la cystine, le chitosane et ses dérivés, les céramides, la biotine, les oligo-éléments, la glycérine, les hydrolysats de protéines, les phospholipides, les agents hydratants. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut être préparée par l'homme du métier sur la base de ses connaissances générales et selon l'état de la technique.

La composition selon l'invention peut se présenter sous la forme d'un produit à appliquer sur les ongles, tel qu'un vernis, une base ou une base de soin.

Les essais de comportement sous frottement indiquent une amélioration de la résistance à l'usure. Les essais de traction monotone et de viscoélasticimétrie indiquent une plus grande aptitude à dissiper l'énergie, par déformation ou par friction interne, d'où il résulte un plus grand pouvoir amortissant, et par conséquent une meilleure résistance à l'écaillage.

L'invention est illustrée plus en détail dans les exemples suivants.

**Exemple 1 :**

On prépare un vernis à ongles ayant la composition suivante (% en poids) :

| . nitrocellulose | 15 % |
|---|---|
| . plastifiant et résine | 16 % |
| . acide silicique colloïdal (AEROSIL 200 de DEGUSSA) | 1 % |
| . solvant (acétates d'éthyle et de butyle) qsp | 100 % |

On obtient un vernis de texture adéquate, qui s'applique aisément sur l'ongle. Il permet, après séchage, l'obtention d'un film lisse et homogène.

**Exemple 2 :**

On prépare un vernis à ongles ayant la composition suivante (% en poids) :

| . pigment | 0,5 % |
|---|---|
| . nitrocellulose | 15 % |
| . plastifiant et résine | 10 % |
| . acide silicique colloïdal (Aerosil 200 de DEGUSSA) | 1 % |
| . solvant (acétates d'éthyle et de butyle) qsp | 100 % |

On obtient un vernis de texture adéquate, qui s'applique aisément sur l'ongle. Il permet, après séchage, l'obtention d'un film lisse et homogène.

## Exemple 3 : Etude du comportement du vernis selon l'invention (exemple 1) par rapport à deux vernis témoins

. Vernis-témoin 1

On prépare un vernis à ongles ayant la composition suivante (% en poids) :

| | |
|---|---|
| . nitrocellulose | 15 % |
| . plastifiant et résine | 16 % |
| . solvant (acétates d'éthyle et de butyle) | qsp 100 % |

. Vernis-témoin 2

On prépare un vernis à ongles ayant la composition suivante (% en poids) :

| | |
|---|---|
| . nitrocellulose | 15 % |
| . plastifiant et résine | 16 % |
| . bentone | 1 % |
| . solvant (acétates d'éthyle et de butyle) | qsp 100 % |

## A) Evaluation du comportement sous frottement :

On suit la perte de masse au cours du temps d'échantillons de vernis testés sur abrasimètre TABER suivant la norme ASTMD 4060-90. On en déduit la vitesse de perte de masse. On mesure également la perte de brillance de chacun des échantillons après 60 mn de ce traitement. Les résultats de ces mesures sont donnés dans le tableau suivant :

| Composition | vitesse de perte de masse (mg/min) | % de perte de brillance après 60 min d'essai (asymptote) |
|---|---|---|
| exemple 1 | 0,62 - 0,53 | 58 - 52 |
| témoin 2 | 0,74 - 0,77 | 67 - 66 |

Les données de ce tableau sont des mesures effectuées sur différents échantillons.
Ces résultats montrent une résistance à l'usure supérieure pour le vernis selon l'invention, par rapport à celle des vernis témoins.

## B) Evaluation de la viscoélasticimétrie :

Les essais de viscoélasticimétrie permettent d'accéder au module complexe $E^* = E' + iE''$ des matériaux testés en fonction de la fréquence (0,1 à 20 Hz) de la sollicitation sinusoïdale imposée et de la température (- 150° à 150°C).
De ces mesures, on déduit les modules dynamiques E', E'' et le pouvoir amortissant : $tg\delta = E''/E'$ .
Les essais sont réalisés sur un appareil DMTA de Polymer Laboratories, sur des échantillons d'environ 100 $\mu$m d'épaisseur, 5 mm de largeur et 10 mm de longueur. On impose une sollicitation de traction.
Les échantillons subissent une force statique de 0,01N à laquelle se superpose un déplacement sinusoïdal de $\pm$ 8 $\mu$m.
On travaille ainsi dans le domaine linéaire, sous de faibles niveaux de déformation.

Le résultat de ces mesures est donné dans le tableau suivant.

| Fréquence (Hz) | 0,1 | 1 | 5 | 20 |
|---|---|---|---|---|
| tg$\delta$ | | | | |
| exemple 1 | 0,91 | 1,09 | 1,04 | 0,95 |
| témoin 1 | 0,79 | 0,81 | 0,72 | 0,61 |
| témoin 2 | 0,84 | 0,81 | 0,69 | 0,60 |

Ces essais montrent un pouvoir amortissant supérieur des produits selon l'invention par rapport aux vernis de l'art antérieur.

**Revendications**

1. Utilisation de l'acide silicique colloïdal en tant qu'agent amortissant dans une composition de vernis ou de base de soin pour ongles comprenant au moins une matière filmogène et au moins un solvant organique et/ou aqueux.

2. Utilisation selon la revendication 1 pour améliorer la tenue du vernis ou de la base, et/ou sa résistance à l'usure.

3. Utilisation selon la revendication 1 ou la revendication 2 pour augmenter la durée de vie du film de vernis à ongles ou de base de soin.

4. Utilisation selon l'une des revendications précédentes, dans laquelle l'acide silicique colloïdal est choisi parmi les silices traitées en surface, les silices pyrogénées hydrophiles et/ou les silices pyrogénées hydrophobes.

5. Utilisation selon l'une des revendications précédentes, dans laquelle l'acide silicique colloïdal est présent en une quantité de 0,05 % à 5 % en poids, de préférence 0,5 - 2 % en poids, par rapport au poids total de la composition.

6. Utilisation selon l'une des revendications précédentes, caractérisée en ce que la composition de base de soin ou de vernis est une composition en milieu solvant organique.

7. Utilisation selon la revendication 6, caractérisée en ce que la matière filmogène est choisie parmi les résines alkydes, acryliques et/ou vinyliques, les polyuréthannes et les polyesters, les celluloses et dérivés cellulosiques telles que la nitrocellulose, et les résines résultant de la condensation de formaldéhyde avec une arylsulfonamide, et leurs mélanges.

8. Utilisation selon l'une des revendications 6 à 7, caractérisée en ce que le solvant organique est choisi parmi le toluène, le xylène, l'acétate d'éthyle, l'acétate de butyle, les cétones, les éthers de glycol, les esters, l'éthanol, l'isopropanol et le butanol, et leurs mélanges.

9. Utilisation selon l'une des revendications 1 à 5, caractérisée en ce que la base de soin ou le vernis à ongles est une composition en milieu aqueux.

10. Utilisation selon la revendication 9, caractérisée en ce que la composition comprend des particules de polymère filmogène en dispersion choisies parmi les polyuréthannes, les polyesters-polyuréthannes, les polyéther-polyuréthannes, les polymères radicalaires de type acrylique, acrylique styrène et acrylique vinylique, les polyesters, les résines alkydes, seuls ou en mélange.

11. Utilisation selon l'une quelconque des revendications précédentes, caractérisée en ce que la dispersion comprend également un polymère associatif de type polyuréthanne ou une gomme naturelle telle que la gomme xanthane.

12. Utilisation selon l'une quelconque des revendications précédentes, caractérisée en ce que la composition présente un taux de matière sèche de 25 - 45 % en poids.

13. Utilisation selon l'une quelconque des revendications précédentes, caractérisée en ce que la composition comprend également un agent plastifiant choisi parmi les citrates, les phtalates, les esters et/ou le camphre en une quantité de 5 - 30 % en poids par rapport au poids de la composition.

**Claims**

1. Use of colloidal silicic acid as cushioning agent in a nail varnish or care base composition including at least one film-forming material and at least one organic and/or aqueous solvent.

2. Use according to Claim 1, for improving the behaviour of the varnish or of the base and/or its resistance to wear.

3. Use according to Claim 1 or Claim 2, for increasing the lifetime of the film of nail varnish or of care base.

4. Use according to one of the preceding claims, in which the colloidal silicic acid is chosen from surface-treated silicas, hydrophilic pyrogenic silicas and/or hydrophobic pyrogenic silicas.

5. Use according to one of the preceding claims, in which the colloidal silicic acid is present in a quantity of 0.05 % to 5 % by weight, preferably 0.5 - 2% by weight, relative to the total weight of the composition.

6. Use according to one of the preceding claims, characterized in that the care base or varnish composition is a composition in an organic solvent medium.

7. Use according to Claim 6, characterized in that the film-forming material is chosen from alkyd, acrylic and/or vinyl resins, polyurethanes and polyesters, celluloses and cellulose derivatives such as nitrocellulose, and resins resulting from the condensation of formaldehyde with an arylsulphonamide, and mixtures thereof.

8. Use according to either of Claims 6 and 7, characterized in that the organic solvent is chosen from toluene, xylene, ethyl acetate, butyl acetate, ketones, glycol ethers, esters, ethanol, isopropanol and butanol, and mixtures thereof.

9. Use according to one of Claims 1 to 5, characterized in that the nail care base or varnish is a composition in an aqueous medium.

10. Use according to Claim 9, characterized in that the composition includes particles of film-forming polymer in dispersion, which are chosen from polyurethanes, polyester-polyurethanes, polyether-polyurethanes, radical polymers of acrylic, styrene acrylic and vinyl acrylic type, polyesters and alkyd resins, alone or mixed.

11. Use according to any one of the preceding claims, characterized in that the dispersion also includes an associative polymer of polyurethane type or a natural gum such as xanthan gum.

12. Use according to any one of the preceding claims, characterized in that the composition has a solids content of 25 - 45 % by weight.

13. Use according to any one of the preceding claims, characterized in that the composition also includes a plasticizing agent chosen from citrates, phthalates, esters and/or camphor in a quantity of 5 - 30 % by weight relative to the weight of the composition.

**Patentansprüche**

1. Verwendung von kolloidaler Kieselsäure als dämpfendes Mittel in Nagellackzusammensetzungen oder Grundmassenzusammensetzungen zur Pflege der Nägel, die mindestens einen Filmbildner und mindestens ein organisches und/oder wässeriges Lösungsmittel enthalten.

2. Verwendung nach Anspruch 1 zur Verbesserung der Haftung des Lacks oder der Grundmasse und/oder ihrer Strapazierfähigkeit.

3. Verwendung nach Anspruch 1 oder Anspruch 2 zur Erhöhung der Lebensdauer des Nagellackfilms oder des Films der Pflegegrundmasse.

4. Verwendung nach einem der vorhergehenden Ansprüche, wobei die kolloidale Kieselsäure unter oberflächenbehandelten Siliciumdioxiden, hydrophilen pyrogenen Siliciumdioxiden und/oder hydrophoben pyrogenen Siliciumdioxiden ausgewählt ist.

5. Verwendung nach einem der vorhergehenden Ansprüche, wobei die kolloidale Kieselsäure in einem Mengenanteil von 0,05 bis 5 Gew.-%, vorzugsweise 0,5 bis 2 Gew.-%, bezogen auf das Gesamtgewicht der Zusammensetzung, vorliegt.

6. Verwendung nach einem der vorhergehenden Ansprüche, dadurch gekennzeichnet, daß es sich bei der Pflegegrundmassenzusammensetzung oder Lackzusammensetzung um eine Zusammensetzung in einem organischen Lösungsmittelmedium handelt.

7. Verwendung nach Anspruch 6, dadurch gekennzeichnet, daß der Filmbildner unter Alkydharzen, Acrylverbindungen und/oder Vinylverbindungen, Polyurethanen und Polyestern, Cellulose und Cellulosederivaten, wie Nitrocellulose, und Harzen, die das Ergebnis der Kondensation von Formaldehyd mit einem Arylsulfonamid sind, sowie den Gemischen dieser Verbindungen ausgewählt ist.

8. Verwendung nach Anspruch 6 oder 7, dadurch gekennzeichnet, daß das organische Lösungsmittel unter Toluol, Xylol, Ethylacetat, Butylacetat, Ketonen, Glykolethern, Estern, Ethanol, Isopropanol und Butanol sowie den Gemischen dieser Verbindungen ausgewählt ist.

9. Verwendung nach einem der Ansprüche 1 bis 5, dadurch gekennzeichnet, daß die Pflegegrundmasse oder der Nagellack eine Zusammensetzung in wässerigem Medium ist.

10. Verwendung nach Anspruch 9, dadurch gekennzeichnet, daß die Zusammensetzung Partikel eines filmbildenden Polymers in Dispersion enthält, das unter Polyurethanen, Polyester-Polyurethanen, Polyether-Polyurethanen, durch radikalische Polymerisation hergestellten Polymeren vom Typ Acryl, Acryl-Styrol und Acryl-Vinyl, Polyestern und Alkydharzen ausgewählt ist, die allein oder im Gemisch vorliegen.

11. Verwendung nach einem der vorhergehenden Ansprüche, dadurch gekennzeichnet, daß die Dispersion ferner ein assoziatives Polymer vom Polyurethantyp oder ein natürliches Gummi, wie Xanthangummi, enthält.

12. Verwendung nach einem der vorhergehenden Ansprüche, dadurch gekennzeichnet, daß die Zusammensetzung einen Trockensubstanzgehalt von 25 bis 45 Gew.-% aufweist.

13. Verwendung nach einem der vorhergehenden Ansprüche, dadurch gekennzeichnet, daß die Zusammensetzung ferner einen Weichmacher enthält, der ausgewählt ist unter Citraten, Phthalaten, Estern und/oder Campher und in einem Mengenanteil von 5 bis 30 Gew.-%, bezogen auf das Gewicht der Zusammensetzung, vorliegt.